# FASCICULE DE BREVET EUROPEEN

(11) **EP 3 270 139 B1**
(45) Date de publication et mention de la délivrance du brevet: **26.08.2020**
(21) Numéro de dépôt: 17177951.5
(22) Date de dépôt: 26.06.2017
(51) Int. Cl.: C12Q 1/6869, G01N 15/12, G01N 27/327, G01N 33/487

(54) **PROCÉDÉ DE DÉTECTION ÉLECTRIQUE DE PEPTIDES, PROTÉINES ET AUTRES MACROMOLÉCULES**
ELEKTRISCHES DETEKTIONSVERFAHREN VON PEPTIDEN, PROTEINEN UND ANDEREN MAKROMOLEKÜLEN
METHOD FOR ELECTRICALLY DETECTING PEPTIDES, PROTEINS AND OTHER MACROMOLECULES

(30) Priorité: 24.06.2016 FR 1601007
(43) Date de publication de la demande: 17.01.2018
(73) Titulaire: EXCILONE, 78990 Elancourt (FR); CY Cergy Paris Université, 95501 Cergy Pontoise (FR); Université Evry Val d'Essonne, 91025 Evry Cedex (FR); ASSISTANCE PUBLIQUE-HOPITAUX DE PARIS, 75010 Paris (FR)
(72) Inventeur: PELTA, Juan, 78990 Elancourt (FR); OUKHALED, Abdelghani, 78990 Elancourt (FR); PIGUET, Fabien, 78990 Elancourt (FR); OULDALI, Hadjer, 78990 Elancourt (FR); KRUPOVA, Zuzana, 78990 Elancourt (FR); DEFRENAIX, Pierre, 78990 Elancourt (FR); MANIVET, Philippe, 78990 Elancourt (FR)
(74) Mandataire: Célanie, Christian

(56) Documents cités:
- CHAN CAO ET AL: "Discrimination of oligonucleotides of different lengths with a wild-type aerolysin nanopore", NATURE NANOTECHNOLOGY, vol. 11, no. 8, 25 avril 2016 (2016-04-25) , pages 713-719, XP055362275, GB ISSN: 1748-3387, DOI: 10.1038/nnano.2016.66 & Chan Cao ET AL: "Discrimination of oligonucleotides of different lengths with a wild-type aerolysin nanopore: Supplementary information", NATURE NANOTECHNOLOGY, vol. 11, no. 8, 19 avril 2016 (2016-04-19) , pages 713-718, XP055431724, GB ISSN: 1748-3387, DOI: 10.1038/nnano.2016.66
- YONG WANG ET AL: "Nanopore Sensing of Botulinum Toxin Type B by Discriminating an Enzymatically Cleaved Peptide from a Synaptic Protein Synaptobrevin 2 Derivative", ACS APPLIED MATERIALS AND INTERFACES, vol. 7, no. 1, 14 janvier 2015 (2015-01-14), pages 184-192, XP055362496, US ISSN: 1944-8244, DOI: 10.1021/am5056596 & Yong Wang ET AL: "Nanopore sensing of botulinum toxin type B by discriminating an enzymatically cleaved Peptide from a synaptic protein synaptobrevin 2 derivative: Supporting Information", Journal of Technology, 29 décembre 2014 (2014-12-29), pages S1-S11, XP055431748, Extrait de l'Internet: URL:http://pubs.acs.org/doi/suppl/10.1021/ am5056596/suppl_file/am5056596_si_001.pdf [extrait le 2017-12-04]
- SUZUKI ET AL: "Expression of genes encoding antimicrobial and bradykinin-related peptides in skin of the stream brown frog Rana sakuraii", PEPT, ELSEVIER, AMSTERDAM, NL, vol. 28, no. 3, 7 février 2007 (2007-02-07), pages 505-514, XP005877253, ISSN: 0196-9781, DOI: 10.1016/J.PEPTIDES.2006.10.016
- JEFF NIVALA ET AL: "Discrimination among Protein Variants Using an Unfoldase-Coupled Nanopore", ACS NANO, vol. 8, no. 12, 23 décembre 2014 (2014-12-23), pages 12365-12375, XP055241720, US ISSN: 1936-0851, DOI: 10.1021/nn5049987
- FABIEN PIGUET ET AL: "Electroosmosis through [alpha]-Hemolysin That Depends on Alkali Cation Type", JOURNAL OF PHYSICAL CHEMISTRY LETTERS, vol. 5, no. 24, 18 décembre 2014 (2014-12-18), pages 4362-4367, XP055362464, US ISSN: 1948-7185, DOI: 10.1021/jz502360c
- ABDELGHANI OUKHALED ET AL: "Sensing Proteins through Nanopores: Fundamental to Applications", ACS CHEMICAL BIOLOGY, vol. 7, no. 12, 21 décembre 2012 (2012-12-21), pages 1935-1949, XP055362448, US ISSN: 1554-8929, DOI: 10.1021/cb300449t
- MORDJANE BOUKHET ET AL: "Probing driving forces in aerolysin and [alpha]-hemolysin biological nanopores: electrophoresis versus electroosmosis", NANOSCALE, vol. 8, no. 43, 3 octobre 2016 (2016-10-03), pages 18352-18359, XP055431375, United Kingdom ISSN: 2040-3364, DOI: 10.1039/C6NR06936C
- Bruno P Meloni ET AL: "Poly-Arginine and Arginine-Rich Peptides are Neuroprotective in Stroke Models", Journal of Cerebral Blood Flow & Metabolism, vol. 35, no. 6, 11 February 2015 (2015-02-11), pages 993-1004, XP055415410, US ISSN: 0271-678X, DOI: 10.1038/jcbfm.2015.11
- STEFUREAC RADU ET AL: "Transport of alpha-helical peptides through alpha-hemolysin and aerolysin pores", BIOCHEMISTRY, AMERICAN CHEMICAL SOCIETY, US, vol. 45, no. 30, 1 August 2006 (2006-08-01), pages 9172-9179, XP002503446, ISSN: 0006-2960, DOI: 10.1021/BI0604835

## Description

Le secteur technique de la présente invention est celui de la détection électrique de molécules en mélange.

Le principe de la détection électrique du transport de molécules à travers un nanopore qui peut être un canal protéique ou un nanotube inséré dans une membrane lipidique ou un trou nanométrique percé dans une membrane solide est bien connu.

La membrane est soumise à une différence de potentiel qui induit un courant ionique à travers le nanopore en présence d'une solution d'électrolytes. Le passage d'une molécule à travers le nanopore, ou l'interaction de la molécule avec le nanopore, induit une chute du courant mesurable. La profondeur de cette chute de courant et sa durée dépendent, en particulier, de la conformation, de la taille, de la séquence et de la nature chimique de la molécule.

Cette sensibilité a été utilisée pour faire du séquençage ultra-rapide d'ADN (Branton et al, 2008, Nat. Biotech ou Karlsson et al, 2015, Scientific reports).

Une autre application très prometteuse concerne la possibilité de déterminer la taille ou la masse d'une molécule ou d'un mélange de molécules donc de faire de la spectrométrie de masse à l'aide d'un nanopore couplé à un système de détection électrique ultrasensible.

Il a été montré la séparation d'un mélange de polymères synthétiques (Roberston et al, 2007, PNAS ; Reiner et al, 2010, PNAS ; Baaken et al, 2011, ACS Nano ; Baaken et al, 2014, ACS Nano) dont une taille de chaîne correspond à une signature électrique de blocage de courant. Il a été également observé récemment la séparation de nucléotides entre 2 et 10 monomères, pour seulement un polydésoxyadénosine pure, à travers un nanopore d'aérolysine (Cao et al, 2016, Nat. nanotech).

L'utilisation du nanopore aérolysine est bien connue et Wilmsen et al 1990 ont montré que l'aérolysine forme un canal sensible au courant électrique dans une bicouche lipidique.

Dans le document de Wang, Y., et al (APPLIED MATERIALS AND INTERFACES, vol. 7, no. 1, 14 janvier 2015 (2015-01-14), pages 184-192), on a étudié le pouvoir de nanopore d'aérolysine à discriminer un peptide de 18 acide aminés coupé par une enzyme.

Dans le document Stefureac et al, 2006, on s'est intéressé au transport de peptides à travers des pores d'aérolysine. On a observé des peptides de différentes longueurs, la différence de temps de blocage de courant mais sans aboutir à une résolution fine à l'échelle d'un acide aminé.

Dans les documents Pastoriza-Gallego et al, JACS 2011 et Payet et al, Anal. Chem. 2012, on s'est intéressé à la dynamique des protéines non repliées (conformations) à travers un nanopore d'aérolysine. Cette étude a porté essentiellement sur la détermination des conformations de grandes protéines (>370 acides aminés) avec l'aérolysine associées à des durées différentes de blocage de courant. Mais, aucune résolution fine n'a été proposée pour distinguer deux peptides ou deux protéines ne différant que par un seul acide aminé.

L'examen de l'abondante littérature disponible dans ce domaine montre que des difficultés existent pour séparer soit des petites chaînes soit des grandes chaînes polypeptidiques, séparation qui n'a pas été résolue à ce jour. La seule résolution fine pour visualiser les énantiomères aromatiques (Tryptophane, Phénylalanine, Tyrosine) a été possible après modification chimique d'un pore d'alpha-hemolysine (Boersma and Bayley, 2012, Angew. Chem. Int. Ed).

Les inventeurs ont donc imaginé l'utilisation d'un canal protéique aérolysine, dans des conditions physico-chimiques précises, afin d'isoler les petites chaînes de quelques monomères sans recourir à des modifications chimiques coûteuses et complexes du nanopore d'aérolysine.

Concernant les peptides ou protéines, de nombreux travaux ont été publiés sur la détermination macroscopique de la taille d'une protéine en utilisant le temps de transport (durée de blocage de courant) ou la profondeur de blocage de courant avec des nanopores solides (Han et al, 2006, APL ; Fologea et al, 2007, APL ; Cressiot et al, 2011, ACS Nano ; Plesa et al, 2013, NanoLetters, Freedman et al, 2013, Scientific reports ; Larkin et al, 2014, BJ, Waduge et al, 2017) ou avec des nanocapillaires en verre (Li et al, 2013, ACS Nano ; Steinbock et al, 2014, Nanoscale). Si ces approches permettent de séparer des protéines natives en fonction de leur taille, il faut cependant que les protéines diffèrent d'au moins 4kDa pour les petites chaînes et de plus de 50 kDa pour les grandes chaînes (Li et al, 2013, ACS Nano ; Steinbock et al, 2014).

A ce jour, on n'a jamais montré la possibilité de faire de la séparation de peptides en fonction de la taille et de la masse en utilisant les nanopores d'aérolysine avec une résolution permettant de déterminer l'ajout d'un acide aminé dans la chaîne.

C'est justement l'objet de l'invention que de proposer l'utilisation d'un nanopore d'aérolysine pour la détection électrique de peptides et de protéines distincts d'au moins un acide aminé et d'autres macromolécules comme les polysaccharides ou les polymères synthétiques ou naturels présents dans une préparation où le dit nanopore est inséré dans une membrane lipidique que l'on soumet à une différence de potentielle comprise entre -60mV et -10mV, dans un milieu réactionnel comprenant une solution d'électrolyte halogénure de métal alcalin ayant une concentration inférieure comprise entre 2M et 5M et une température comprise entre 3°C et 33°C, et où la dite utilisation a pour objectif de différencier les dits peptides et protéines, en fonction de leur longueur et leur masse et de leur séquence.

Selon une caractéristique de l'invention, l'halogénure de métal alcalin est un chlorure de métal alcalin et représenté par LiCl, KCl ou NaCl.

Selon encore une autre caractéristique de l'invention, la différence de potentiel appliquée à la membrane est avantageusement de -29 mV.

Une utilisation particulièrement avantageuse de la présente méthode décrite précédemment réside dans la caractérisation de la dégradation enzymatique d'un échantillon de peptides, de protéines, de polysaccharides ou de polymères synthétiques par détermination des produits de dégradation.

Une autre utilisation particulièrement avantageuse de la présente méthode décrite précédemment réside dans la caractérisation des modifications chimiques naturelles ou synthétiques de molécules natives telles qu'une protéine, un peptide, un métabolite, un médicament.

Une autre utilisation encore particulièrement avantageuse de la présente méthode décrite précédemment réside dans la caractérisation l'activité enzymatique spécifique et molaire d'une enzyme dans une cellule unique procaryote ou eucaryote.

Une utilisation particulièrement avantageuse décrite dans la présente demande réside dans la caractérisation pour identifier et quantifier des molécules dites « métabolites » appartenant à des voies métaboliques ou de signalisations cellulaires.

La présente méthode décrite précédemment décrit également le produit peptidique obtenu par l'utilisation d'un nanopore constitué d'un peptide unique tel le peptide RR10.

Un tout premier avantage de l'invention réside dans la grande sensibilité et la haute résolution de la technique qui permet de différencier des molécules très proches l'une de l'autre, différentes d'un monomère.

Un autre avantage de l'utilisation de la présente méthode décrite précédemment réside dans la séparation des séquences différentes de peptides ou protéines. Les peptides, protéines, polysaccharides ou polymères synthétiques sont présents dans une préparation présentée comme un mélange complexe, biologique ou commercial pur.

Ainsi, la méthode décrite précédemment permet une résolution allant jusqu'à un acide aminé, un sucre ou un monomère de différence entre deux molécules.

Un autre avantage de la présente méthode décrite précédemment réside dans le fait que cette grande sensibilité permet d'effectuer une analyse à partir d'un échantillon en très faible quantité.

Un autre avantage de la présente méthode réside dans les nombreuses applications offertes comme la réalisation d'un séquençage des peptides.

Un autre avantage de la présente méthode décrite précédemment réside dans la possibilité d'effectuer une certification de qualité 100% pure d'un peptide ou d'une molécule synthétisée.

Un autre avantage de la présente méthode décrite précédemment réside dans la possibilité d'identifier et de quantifier des médicaments dans tous les fluides biologiques, dans une cellule unique.

Un autre avantage encore de la présente méthode décrite précédemment réside dans la capacité d'identification d'un peptide pour le diagnostique médical biologique.

Un autre avantage encore de la présente méthode décrite précédemment réside dans la possibilité de conserver très longtemps un peptide ou un mélange de peptides, une protéine par lyophilisation pour obtenir une poudre.

On peut alors insérer cette poudre en la protégeant avec un film pour l'utiliser comme code moléculaire à titre de marquage afin de l'identifier de façon certaine pour montrer son origine ou son unicité, telle l'origine de production d'un produit, la certification d'origine.... Ensuite, le contrôle se fera en mettant la poudre dans une solution tampon. La conservation à température ambiante d'une solution est de quelques jours à quelques mois, par exemple à 4°C la durée est de plusieurs mois, à -20°C la durée est de plusieurs années.

On entend par mélange de peptides bien défini un mélange contenant par exemple 95% d'un peptide à 10 AA et 5% à 8 AA. Chacun des lots de ces peptides pouvant être modifié chimiquement (phosphorylation, halogénation, etc.), ce mélange étant une préparation présentée comme un mélange complexe, biologique ou commercial pure.

Il s'agirait d'un code moléculaire invisible et donc discret qui pourrait être lu, en remettant en suspension le mélange de peptides et en le décryptant avec la technologie décrite ici.

D'autres caractéristiques, avantages et détails de la présente méthode décrite précédemment seront mieux compris à la lecture du complément de description qui va suivre de modes de réalisation donnés à titre d'exemple en relation avec des dessins sur lesquels :
- la figure 1 représente un exemple de réalisation d'un dispositif pour mettre en œuvre l'invention,
- la figure 2 représente la variation de la différence de potentiel en fonction du temps d'élution correspondant à la détection d'un peptide,
- la figure 3 représente l'analyse d'un mélange de six peptides,
- la figure 4 représente les résultats d'analyse d'un échantillon de peptides du commerce,
- la figure 5 illustre la dégradation enzymatique d'un échantillon de peptides de 10 acides aminés par la trypsine.
- la figure 6 illustre la détection des peptides en fonction de la variation de la différence de potentiel,
- la figure 7 montre la discrimination de peptides de 10 acides aminés de long, présentant des séquences différentes,
- la figure 8 montre la détection d'une activité enzymatique à partir d'une cellule ou vésicule unique, et
- la figure 9 représente une variante du processus selon la figure 7.

Comme il ressort de ce qui précède, il est possible maintenant, suivant l'utilisation et le procédé décrits précédemment de détecter à partir d'une mesure électrique, des peptides individuels et de distinguer des peptides de tailles différentes avec une résolution d'un seul acide aminé. Il en est de même de la détection d'un très grand nombre de molécules comme les protéines, les polysaccharides ou les polymères synthétiques ou naturels présents dans une préparation.

Dans la suite de la description ci-après, on va décrire plus particulièrement à des fins d'illustration la mise en œuvre d'un produit vendu dans le commerce renfermant le peptide RR10 donné pur afin de déterminer sa composition exacte.

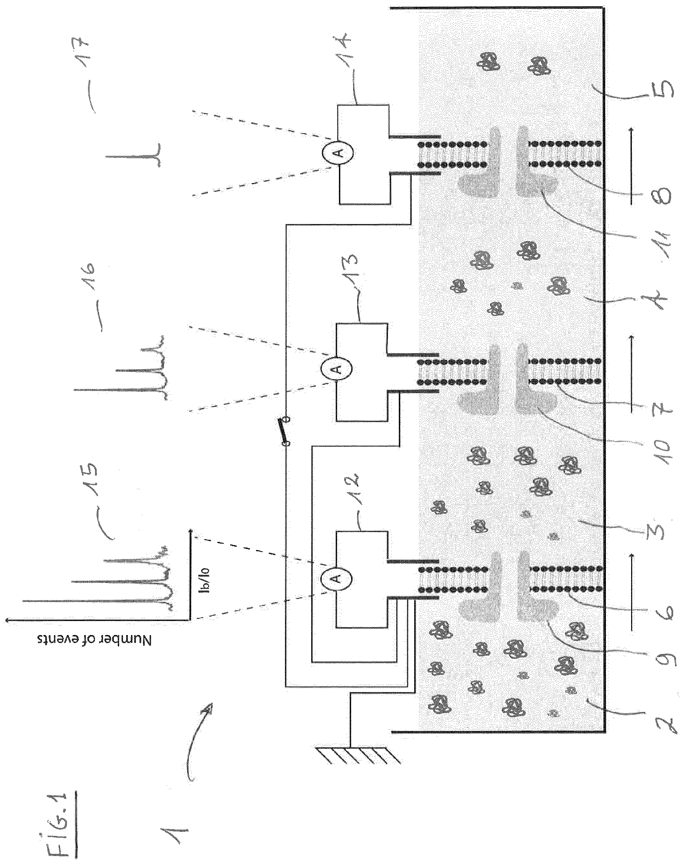

Sur la figure 1, on a décrit un exemple de réalisation d'un dispositif 1 permettant de séquencer un produit commercial ou autre, du type protéines, polysaccharides ou polymères synthétiques ou naturels présents dans une préparation. Il va de soi que pour chaque catégorie de produit les conditions opératoires sont adaptées en fonction des critères de recherche.

Le dispositif 1 comprend par exemple quatre compartiments en série 2, 3, 4 et 5 séparés les uns des autres par trois membranes lipidiques 6, 7 et 8 dans lesquelles les napores d'aérolysine ou nanotubes respectifs 9, 10 et 11 sont insérés.

Les membranes 6, 7 et 8 sont soumises à un courant électrique à l'aide des circuits respectifs 12, 13 et 14. Ainsi, chaque membrane peut être soumise à un niveau de courant selon les conditions opératoires.

Le dispositif 1 permet donc de suivre en temps réel la translocation des molécules dans les différents compartiments 2 à 5.

Bien entendu, chaque circuit électrique 12, 13 ou 14 est relié respectivement à un détecteur électrique permettant de représenter le passage de chaque molécule que renferme le produit à analyser. Dans cet exemple, il s'agit d'un échantillon de peptides. On voit par exemple que la membrane 6 permet de tracer les trois pictogrammes respectifs 15, 16 et 17. Le pictogramme 15 permet d'identifier trois pics correspondant à 3 peptides, le pictogramme 16 deux pics intéressants de deux peptides et le pictogramme 17 un pic unique d'un seul peptide.

La translocation vers le compartiment 5 est arrêtée par l'ouverture du circuit électrique 14 pour prélever le peptide pur présent dans ce compartiment.

Les pictogrammes 15, 16 et 17 représentés sur la figure 1 correspondent à l'analyse d'un produit peptide dont les conditions expérimentales précises sont données ci-après. Il va de soi que le dispositif 1 est opérationnel lorsqu'on procède à l'analyse d'un produit constitué par des protéines, des polysaccharides ou des polymères synthétiques ou naturels. Il va de soi encore que le nombre de pics est fonction de la nature du produit de départ.

On va maintenant s'attacher à l'étude d'un produit commercial donné pur à 96%, le peptide RR10. La connaissance exacte de sa composition et de la nature des impuretés est un élément important en médecine humaine auquel les spécialistes sont attachés.

On a donc effectué à titre d'exemple l'analyse d'un produit vendu dans le commerce, présenté comme étant constitué du peptide RR10 pur à 96% de la manière suivante, afin de déterminer quelle est la composition chimique exacte de ces impuretés.

L'installation est analogue à celle représentée sur la figure 1 éventuellement réduit à deux compartiments. On place alors une membrane lipidique, par exemple la membrane 6 contenant le nanopore d'aérolysine 9, dans un milieu de réaction présentant une concentration en chlorure de lithium de 4M, à une température de 5°C, et on soumet la membrane à une différence de potentiel de -29mV. Grâce à un détecteur électrique extrêmement sensible, on mesure le courant électrique circulant à travers les pores.

La différence de potentiel provoque la circulation d'ions de l'électrolyte entre les deux côtés de la membrane, par exemple dans les compartiments 2 et 3, ce qui crée un courant mesurable. Lorsque des molécules passent à travers le nanotube, elles interrompent le courant. Ces interruptions sont proportionnelles à la taille des molécules, et varient en fonction des conditions de concentration, de pH et de température du milieu de réaction.

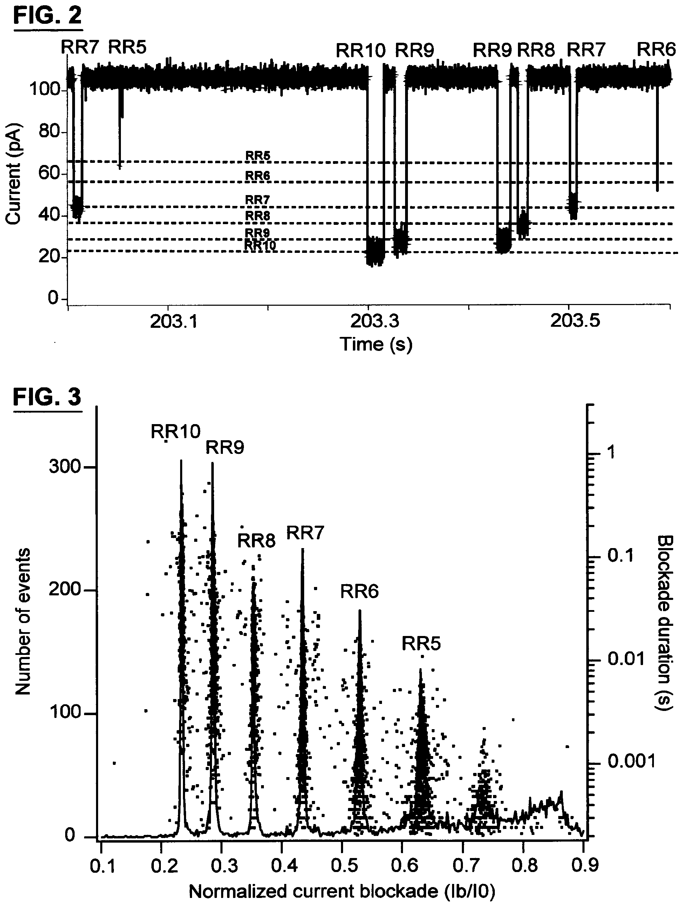

On mesure le courant I en permanence au cours du temps et on voit sur la figure 2 le courant de départ I0 quand les ions circulent sans obstacle et le courant Ib quand un blocage du nanopore est en cours. On peut voir clairement les chutes de courant correspondant aux différents peptides composant le mélange présenté comme pur. On voit que ces impuretés sont des sous-produits de la synthèse du peptide RR10, des peptides incomplets présentant chacun un moment de passage spécifique ainsi qu'une chute de courant caractéristique étalée comme indiqué précédemment de 30 à 68 pA.

Sur la figure 2, on retrouve différentes signatures à des niveaux de courant différents. Ainsi, pour une durée d'expérience de 203,3 s et à une tension de 25 pA, on détecte une chute du courant correspondant au peptide RR10. On voit que la mesure effectuée révèle la présence d'impuretés constituées par les peptides RR9, RR8, RR7, RR6 et RR5 à des valeurs de courant comprises entre 30 pA et 68 pA.

A partir de ces mesures, on a construit à l'échelle de la molécule unique le spectrogramme de masse représenté sur la figure 3. On voit que dans l'échantillon analysé le peptide RR10 donné pour une concentration pure à 96% est toutefois accompagné de plusieurs autres peptides. On a déterminé qu'il s'agissait des peptides RR9 à RR5.

L'utilisation du nanopore permet d'atteindre une sensibilité très élevée qui révèle la présence de fragments peptidiques quantifiables (de l'ordre de 1% du nombre total de molécules détectées, soit quelques dizaines de molécules) dans un échantillon vendu pur à 96%. Jusqu'à ce jour, il était impossible d'identifier ces fragments par l'analyse de spectrométrie de masse classique et par analyse chromatographique HPLC de la composition fournie par le vendeur de cet échantillon.

On comprend tout l'intérêt de la présente méthode décrite précédemment qui permet d'atteindre un niveau de détection de peptides, protéines, polysaccharides et polymères synthétiques et autres molécules impossible avec les autres techniques classiques d'analyse.

Afin d'obtenir une résolution et une sensibilité satisfaisantes, différents facteurs sont à prendre en considération.

On a donc procédé à divers essais en faisant varier les conditions physico-chimiques du milieu de réaction : la concentration en électrolyte, la nature de cet électrolyte, la température du milieu et la différence de potentiel appliquée à la membrane. On a ainsi constaté qu'une augmentation de la concentration en électrolyte et une diminution de la température favorisent la détection des peptides les plus courts et *vice versa.* Il en est de même des autres molécules.

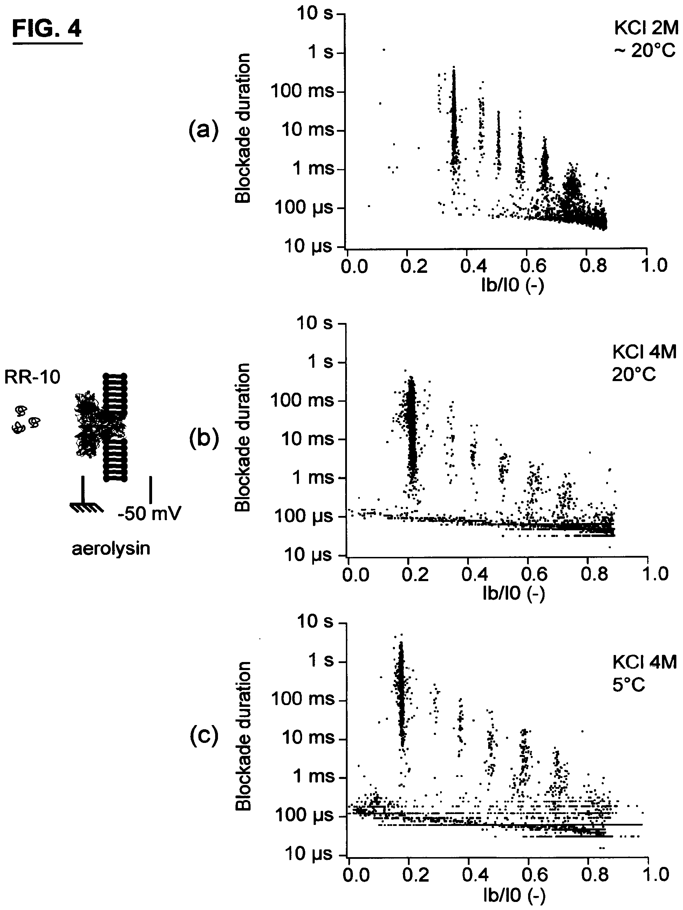

En effet, comme on peut le voir sur les trois graphes de la figure 4, la diminution de la température et l'augmentation de la concentration en électrolyte du mélange de réaction entraînent une meilleure résolution sur les peptides de petite taille. Sur le graphe (a), où la température est de 20°C et la concentration en électrolyte est de 2M, on voit que sur les tailles les plus petites, la résolution est plus faible : les pics ne sont pas nets et on constate une dispersion dense d'événements. Sur les graphes (b) et (c), où la concentration en électrolyte a été augmentée nettement (4M) et où la température est diminuée (5 °C, graphe (c)), on voit bien que la définition pour les valeurs élevées de Ib/I0, c'est-à-dire pour les peptides les plus petits, est meilleure. Les événements sont nettement séparés en deux pics distincts.

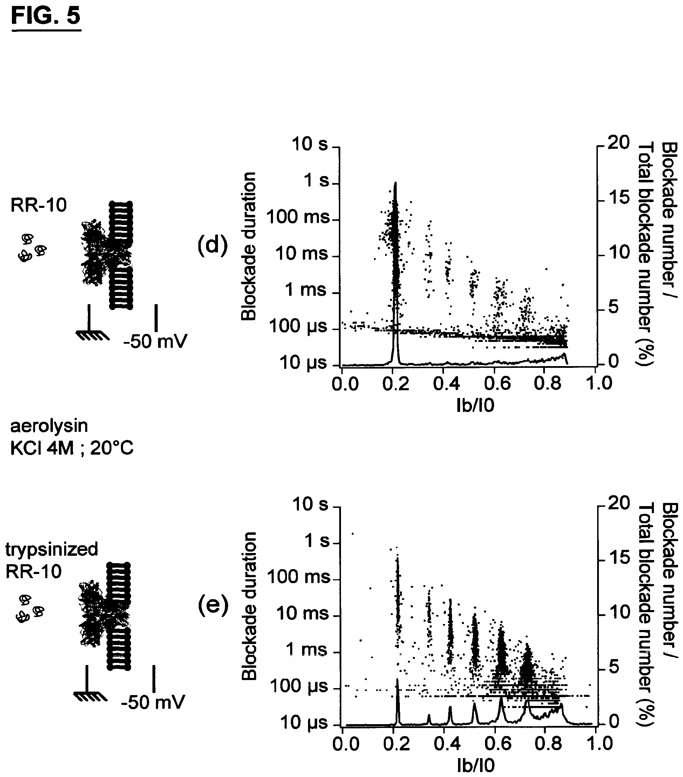

Ainsi, sur la figure 5, on a représenté les résultats obtenus en faisant varier la température et la concentration en électrolyte. On a représenté la durée de blocage de courant en fonction de Ib qui est l'intensité du courant du canal bloqué du nanopore aérolysine et I0 l'intensité du courant du même canal vide du nanopore.

Concernant le graphe (d), la température est de l'ordre de 20 °C et la concentration en électrolyte de 2M. Sur le graphe (e), la température est de 20 °C environ et la concentration en électrolyte est de 4M. Sur la courbe du bas, la température est de 5 °C et la concentration en électrolyte de 4M.

On a déterminé que la concentration de la solution d'électrolyte pourrait être comprise entre 0,5 et 5M et qu'une concentration de 1 M constitue un bon compromis, par exemple avec l'électrolyte LiCl.

On a utilisé différents électrolytes comme par exemple LiCl, NaCl, KCl ou KBr, RbCl, CsCl, KF, ou chlorure d'ammonium, tétraméthylammonium chloride,.... Les résultats obtenus sont globalement du même ordre quel que soit l'électrolyte.

Ainsi, la concentration élevée en électrolyte permet d'étudier des molécules de masse moléculaire plus faible, de l'ordre de deux acides aminés, et de manière surprenante un seul acide aminé. Dans ces conditions, le rapport signal/bruit est meilleur et les valeurs de Ib/I0 sont décalées vers la gauche du spectrogramme. En effet, plus le rapport Ib/I0 se rapproche de 0, plus la molécule concernée est longue. Ce décalage vers la gauche induit une marge à droite du spectrogramme favorisant ainsi la détection de peptides dont la longueur est inférieure à trois acides aminés. Compte tenu de la marge à droite du spectrogramme et de la forte concentration en sel, la détection d'un seul acide aminé est accessible.

Quant à la température, l'étude des trois diagrammes de la figure 4 montre que la position des peptides varie en ordonnée et qu'il est plus intéressant de préférer une température de 5°C et une concentration de 4M. On améliore donc la résolution. De manière générale, la température du milieu réactionnel peut être comprise entre 3 et 33°C.

La différence de potentiel appliquée à la membrane nanopore aérolysine ne semble pas avoir d'effet sur la position des raies figurant sur les graphes de la figure 4. Toutefois, en passant par exemple de -10 mV à -80 mV, on a constaté que la durée moyenne de bouchage du courant dépend du voltage pour l'échantillon de peptides RR10. Le choix d'une valeur de courant dépendra donc de la résolution que souhaite obtenir l'utilisateur. Une différence de potentiel appliquée à la membrane de -29 mV permet d'obtenir une excellente résolution.

Dans ce qui précède, on a mis en œuvre un nanopore aérolysine mais il va de soi que d'autres nanopores peuvent être utilisés. Ainsi, un nanopore ou nanotube du type cyclodextrine peut être mis en œuvre dans le procédé selon l'invention en adaptant sans difficultés les conditions d'utilisation à savoir la température, la concentration en sel et la nature de l'électrolyte.

Les conditions d'analyse de l'échantillon peptide décrit largement ci-dessus sont applicables aux autres échantillons protéines, quant à la nature de l'électrolyte et sa concentration, quant à la température du milieu réactionnel et quant au niveau du courant appliqué au nanopore ou au nanotube. Il va de soi que ces conditions sont adaptées suivant la nature de ces molécules et l'homme du métier dispose des éléments nécessaires pour définir par des simples essais celles applicables aux protéines, aux oligosaccharides ou aux polymères synthétiques ou naturels. A titre d'illustration, on a déterminé que plus la molécule concernée est longue, plus cette longueur induisait un rapport Ib/I0 se rapprochant de 0.

Un autre intérêt de l'invention repose sur les réactions enzymatiques. On sait que de nombreuses réactions enzymatiques sont déterminantes pour doser un produit important pour le diagnostic d'une maladie ou pour un processus industriel. On peut donc, suivant l'invention, suivre la dégradation enzymatique d'un peptide, par exemple par la trypsine, et les constantes cinétiques ont été bien déterminées. Bien évidemment l'homme du métier pourra conduire une dégradation d'une protéine, d'un polysaccharide ou d'un polymère synthétique ou naturel en procédant à des adaptations et quelques essais.

La figure 5 illustre les résultats obtenus en procédant à une analyse suivant l'invention avant (graphe d) et après (graphe e) dégradation enzymatique en mesurant l'intensité Ib du courant du canal bloqué et l'intensité I0 du courant du même canal de l'échantillon donné pur à 96%. Sur ces diagrammes, la raie la plus à gauche représente le peptide RR10, les autres peptides étant sous forme de trace.

Sur le premier diagramme, on voit que, avant dégradation, le peptide RR10 est le constituant majeur de l'échantillon. Après dégradation par la trypsine, on voit que l'appauvrissement en peptide RR10 s'effectue au profit des autres peptides RR9, RR8, RR7, RR6, RR5.

L'utilisation d'un nanopore permet donc de visualiser la dégradation du peptide RR10 par la trypsine, en peptides plus courts : RR9, RR8, RR7, RR6 et RR5. Par conséquent, on peut utiliser ce même procédé pour suivre d'autres processus de dégradation enzymatique de protéines, de polysaccharides, de polymères synthétiques ou naturels ou d'autres macromolécules susceptibles d'intéresser un utilisateur (médicaments, produits industriels ou autres).

Par ailleurs, on distingue nettement les peptides RR10, RR9, RR8, RR7, RR6 et RR5. On peut donc différencier des peptides ne présentant qu'un acide aminé de différence. Cette caractéristique permet un suivi extrêmement fin et spécifique des processus chimiques.

Dans l'exemple, on cite un peptide de 10 acides aminés de long, mais il va de soit que l'invention peut être utilisée avec des peptides plus longs : 20, 50 ou 100 acides aminés, ainsi que sur des macromolécules d'autres types (protéines, polysaccharides, polymères synthétiques ou naturels).

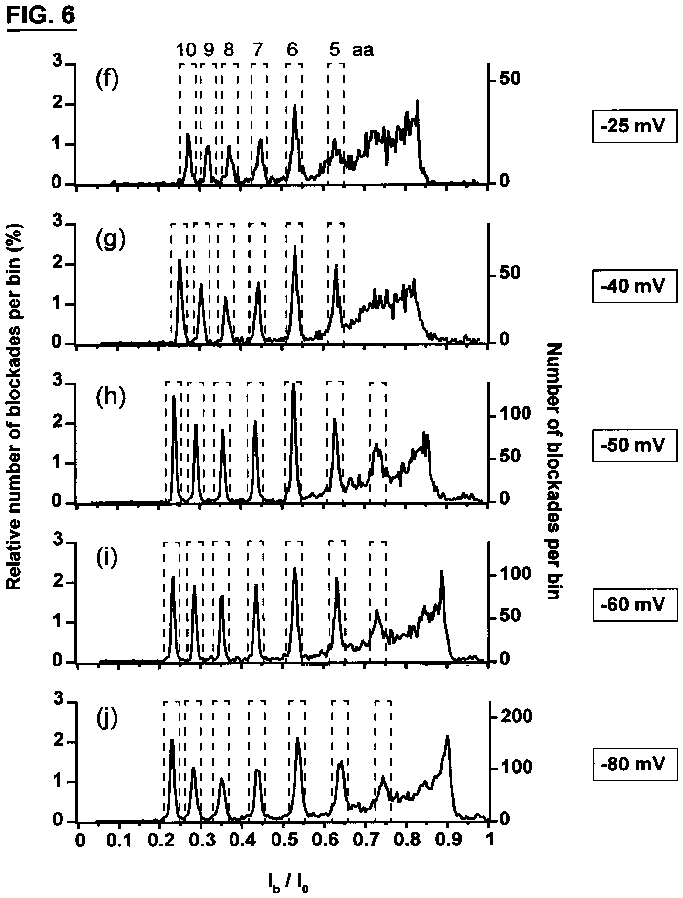

Sur la figure 6, on a représenté 5 histogrammes illustrant l'effet du voltage sur la discrimination des peptides arginine de différentes longueurs. Ces histogrammes représentent les valeurs de courant de blocage Ib/I0 dans le cas de l'interaction du nanopore d'aérolysine avec un mélange équimolaire de peptides d'arginine de différentes longueurs (5, 6, 7, 8, 9 et 10 acides aminés) à -25 mV (a), à -40 mV (b), -50 mV (c), -60 mV (d) et -80 mV (e). Les mesures sont conduites en milieu KCl 4M, à pH 7,5 et à 20 °C.

On note qu'on distingue 6 populations de peptides correspondant aux 6 longueurs de peptides différents de 5 à 10 acides aminés pour un voltage inférieur à -50 mV (graphes f et g). Par contre, pour un voltage supérieur ou égal à -50 mV, on repère une 7^{ème} population représente en pointillé (graphes h, i et j).

Ainsi, le procédé décrit permet une analyse fine d'échantillons de molécules comme les peptides en distinguant un peptide différent d'un autre que par la présence d'un seul acide aminé. De plus, tous les essais de séquençage décrits ci-dessus et validés ont été effectués sur des petites quantités de peptides ou protéines, soit quelques dizaines de molécules par exemple de 2 à 15 dizaines

On a effectué les travaux précédents en s'attachant particulièrement à des échantillons donnés purs à 96% afin d'illustrer l'invention. Mais, on a obtenu la séparation de peptides de différentes tailles lorsque l'échantillon peptidique est totalement logé dans le nanopore et que chaque monomère (acide aminé) contribue au blocage de courant. On comprend qu'il n'y a pas de limite à l'analyse d'échantillons de peptides dès lors que la dimension du nanopore recevant les peptides est ajustée.

Il suffit de recueillir les différentes fractions de peptides suivant un processus analogue à celui mis en œuvre dans les chromatographies afin d'isoler une quantité pure de peptide unique comme cela a été expliqué en relation avec la figure 1.

On a illustré abondamment l'application de la méthode décrite précédemment à l'étude d'un échantillon donné pur du peptide RR10. Il va sans dire que l'invention est mise en œuvre pour caractériser n'importe quel peptide donné pur. Ainsi, on a pu caractériser des peptides de tailles plus importantes tels que les peptides RR20, RR50 ou RR80. Il s'agit donc d'une avancée majeure dans la connaissance précise des échantillons utilisés en médecine.

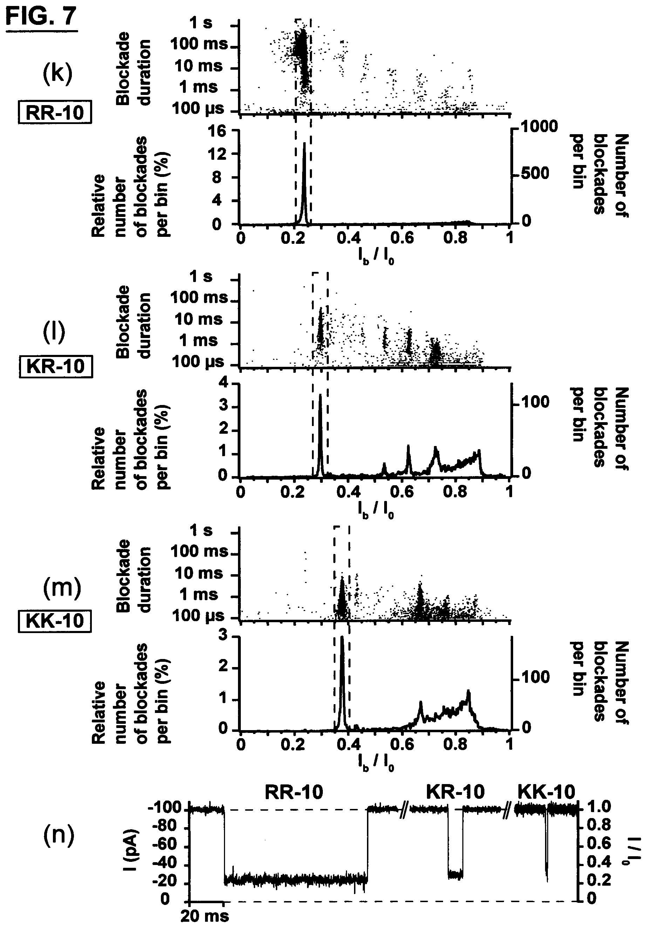

Par ailleurs, la méthode décrite précédemment peut être appliquée à l'étude d'échantillons comprenant plusieurs peptides de même longueur, mais de séquences différentes, comme représenté sur la figure 7.

Pour ce faire, selon le même protocole que décrit précédemment, on a étudié des échantillons de pureté élevée (supérieure à 98%) de peptides d'arginine de 10 acides aminés de long (RR10), représenté sur le graphe k, et de peptides de lysine de 10 acides aminés de long (KK10), représenté sur le graphe l. On voit sur les graphes que les populations majoritaires repérées diffèrent à la fois en terme de valeur du rapport Ib/I0 et en durée moyenne de blocage. Cela révèle que la méthode décrite précédemment permet de différencier des homopeptides de même longueur, mais de séquences différentes.

De plus, on a testé un échantillon de pureté élevée (supérieure à 98%) d'un hétéropeptide composé de 5 acides aminés lysine et 5 acides aminés arginine (KR10), représenté sur le graphe m. On voit clairement sur le graphe m que cette séquence correspond à un profil de blocage du nanopore, en terme de durée et d'intensité, différent des deux autres peptides.

Le graphe n représente le blocage typique créé par ces trois peptides au cours du temps.

La méthode décrite précédemment peut donc également discriminer entre peptides de même longueur, des peptides de séquences différentes, ce qui ouvre des perspectives nouvelles pour l'analyse biologique et médicale.

La méthode décrite précédemment trouve une application particulièrement importante en métabolomique et enzymologie.

Le diagnostic médical biologique doit aujourd'hui évoluer afin de permettre de travailler sur des échantillons précieux et rares ne contenant que quelques cellules ou quelques microvésicules d'intérêt ou encore sur de petits volumes d'échantillon tel que ceux recueillis chez la souris.

Les méthodes classiques de dosage qui utilisent la spectrométrie de luminescence, l'absorbance ou encore la fluorescence, nécessitent de grandes quantités d'échantillon pour obtenir un signal suffisant, distinct du bruit de fond. Par exemple, un minimum de 500 000 cellules sanguines blanches est nécessaire pour le dosage de l'activité des complexes enzymatiques de la chaîne respiratoire. Ces méthodes classiques interdisent par exemple de réaliser un profil enzymatique sur une cellule tumorale circulante isolée du sang.

On peut également utiliser un nanopore conformément à la méthode décrite précédemment pour séparer des séquences différentes de peptides ou de protéines. La séquence d'un peptide conditionne ses propriétés physico-chimiques, qui elles-mêmes conditionnent son passage dans un nanopore. En effet, la nature des acides aminés conditionne la conformation des peptides. Différentes formes de protéines traversent les nanopores à des vitesses différentes. De même, les autres propriétés des protéines (charge, hydrophobie, etc...) influent sur la vitesse et la fréquence de leur passage à travers un nanopore, et donc sur les caractéristiques du blocage de courant qu'elles induisent. Par conséquent, il est possible, grâce à l'utilisation d'un nanopore selon la méthode décrite précédemment d'identifier différentes protéines présentes dans un mélange, différant par leurs tailles mais également par leurs séquences.

Pour ce faire, on place le mélange de protéines à séparer et identifier dans les conditions appropriées et on procède au passage à travers le nanopore. Chaque protéine crée sa signature spécifique en termes de durée de blocage spécifique du courant et de d'amplitude de blocage de courant, présentant donc des propriétés particulières de durée, d'intensité et de moment.

Il est également possible d'utiliser un nanopore selon la présente méthode décrite précédemment pour caractériser les modifications chimiques naturelles ou synthétiques de molécules natives telles qu'une protéine, un peptide, un métabolite, un médicament.

Les modifications chimiques subies par une molécule changent ses caractéristiques : taille, conformation, charge, hydrophobie, etc... Comme déjà expliqué ci-dessus, ce sont des caractéristiques qui influent sur le passage à travers un nanopore et par conséquent sur les blocages de courant provoqués par ce passage.

La technologie nanopore aérolysine mise au point dans la description permet de doser l'activité d'un petit nombre de copies d'enzymes dans une cellule unique, selon un processus industriel, permettant de quantifier l'activité enzymatique d'un faible nombre de molécules d'enzyme qui sont libérées après lyse de la celle d'origine.

Même les méthodes les plus sophistiquées telles que la spectrométrie de masse en tandem demande un minimum de 50 000 cellules. C'est pourquoi les inventeurs se sont orientés vers le développement d'une technologie « nanopore » qui permet de doser l'activité d'un petit nombre de copies d'enzymes dans une cellule unique.

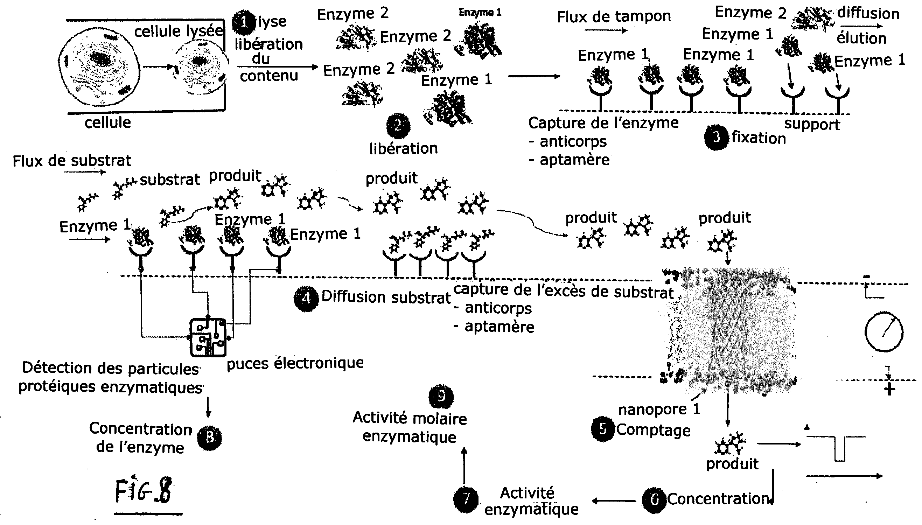

La figure 8 illustre l'exemple d'un possible processus industriel permettant de quantifier l'activité enzymatique d'un faible nombre de molécules d'enzyme qui auraient été libérées après lyse de la celle d'origine. Ce système de détection et de quantification spécifique permet donc de faire sur cellule unique des dosages jusqu'alors impossibles à réaliser. Son utilisation en santé ouvre un champ d'application très large, notamment celui de la médecine personnalisée.

Ce processus est par exemple le suivant et illustré sur la figure 8 qui représente un synoptique du système microfluidique permettant le dosage d'une activité enzymatique spécifique où l'enzyme est amenée à l'entrée du pore par guidage microfluidique.
En 1, la cellule est lysée de manière classique pour libérer son contenu enzymatique. La cellule unique peut être du type procaryote ou eucaryote
En 2, les molécules de l'enzyme d'intérêt et d'autres molécules provenant du contenu de la cellule lysée sont entrainées par l'écoulement d'une solution tampon dans les tubulures du système microfluidique (système permettant de prendre en charge des fluides dont le volume est de 10⁻⁹ à 10⁻¹⁸ litres) jusqu'à une chambre réactionnelle.
En 3, dans la chambre réactionnelle les molécules de l'enzyme d'intérêt sont capturées et fixées sur un support par des sondes spécifiques qui peuvent être des anticorps, des sondes aptamériques, des peptides spécifiques, etc. L'écoulement continu du tampon permet de s'affranchir du phénomène de diffusion. Les autres molécules sont éliminées par diffusion.
En 4, le substrat spécifique de l'enzyme d'intérêt est ensuite injecté et capturé par les enzymes d'intérêt fixées sur le support. Ces enzymes transforment le substrat en produit qui à son tour diffuse dans le tampon. L'excès de substrat est capturé par des sondes spécifiques en aval.
En 5, enfin, les molécules de produits passent dans une série de nanopores aérolysine.
En 7 et 8, chaque passage est détecté et permet de compter chaque molécule et d'en déduire la concentration et l'activité enzymatique. La concentration en enzyme est déterminée par un système électronique.

Cette quantification du nombre de molécules d'enzyme combinée à celle du produit permet en 9 d'obtenir une activité enzymatique molaire de très haute précision.

Selon le même processus, il est possible d'identifier et de quantifier des molécules dites « métabolites », appartenant à des voies métaboliques ou jouant un rôle dans la signalisation cellulaire.

De la même façon, on traite un lysat de cellule pour identifier sa composition. Des substrats enzymatiques ou des ligands particuliers au processus biologique étudié permettent de ne détecter que les molécules impliquées dans les processus en question.

Ces molécules dites métabolites sont produites en général par des enzymes appartenant à des voies biochimiques cellulaires telles que le cycle de KREBS, la voie des pentoses phosphates, etc. Ces métabolites sont très souvent tour à tour substrats et produits des réactions enzymatiques qui s'enchaînent les unes aux autres dans les voies métaboliques cellulaires. Pour la voie des pentoses phosphates, ces métabolites sont par exemple le glucose-6 phosphate, le 6-phosphoglucnate ou encore le glycéraldéhyde-3-phosphate. Dans le cycle de KREBS les métabolites sont à titre d'exemple le citrate, le succinate ou l'alphacétoglutarate. La concentration de ces métabolites varie en fonction de l'homéostasie cellulaire et plus particulièrement en cas de pathologie. Dans une cellule unique ces métabolites sont en quantité non détectables par les techniques de dosages existantes. Outre les métabolites décrits plus haut, la méthode décrite précédemment permet aussi le dosage d'hormones, de neuromédiateurs, de neurotransmetteurs, de molécules présentes dans les voies de signalisation cellulaires ou messagers tels que l'ATP, l'ADP, l'AMPc, l'IP3, l'acide arachidonique.

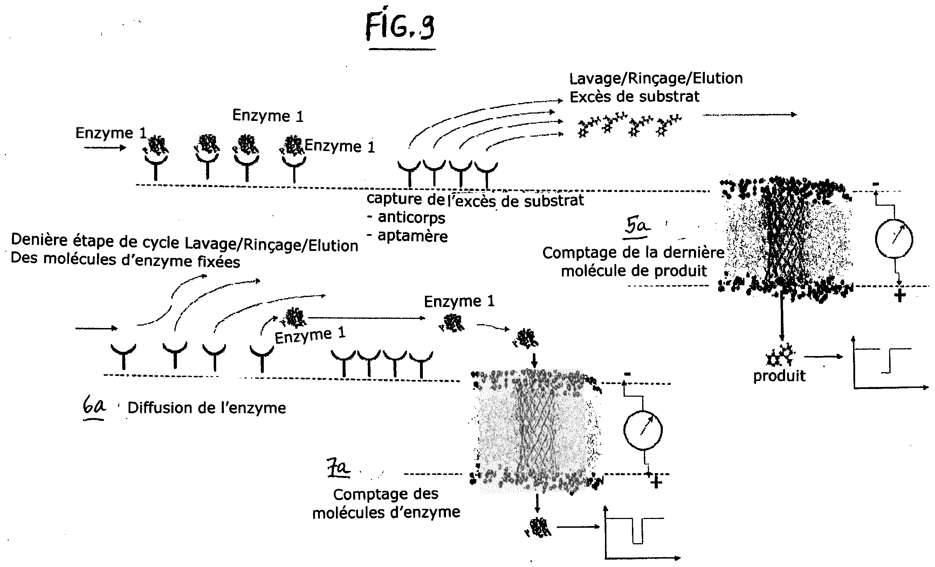

Sur la figure 9, on a représenté une variante de réalisation du système illustré par la figure 8. Le système de détection et de quantification électronique des molécules d'enzyme en 8 peut être remplacé par les nanopore en 5 après avoir élué toutes les molécules de substrat en excès et compté toutes les molécules de produit. Plusieurs cycles de lavages/rinçages seront réalisés de façon ménagée afin de ne pas éluer les molécules d'enzymes accrochées sur leurs sondes spécifiques en 4 avec les molécules de substrat et de produit. Une fois le micro capillaire rincé, l'élution des molécules d'enzyme fixées en 4 est réalisée. Ces molécules d'enzyme sont ensuite comptées en 5 par les nanopores qui ont servi auparavant à compter les molécules de produit.

En 5a, l'excès de substrat est rincé dans un flux de tampon. Le courant de transfert dans le nanopores est coupé. Les conditions d'élution et de rinçage sont telles qu'elles permettent aux molécules d'enzyme de rester fixées sur leurs sondes spécifiques. En 6a, les conditions d'élution sont plus drastiques et permettent aux molécules d'enzyme de quitter leurs sondes spécifiques et de diffuser au travers des nanopores après que l'on ait rétabli le courant de passage.

Les figures ci-dessus 8 et 9 illustrent également le principe de l'invention pour le dosage quantitatif de métabolites et la détermination de l'activité enzymatique molaire de n'importe quelle enzyme.

Il suffit de remplacer le processus des figures 8 et 9 les molécules de protéines enzymatiques par les métabolites et de les simplifier en supprimant l'étape de capture des enzymes.

## Revendications

1. Utilisation d'un nanopore d'aérolysine pour la détection électrique de peptides et de protéines distincts d'au moins un acide aminé dans une préparation où le dit nanopore est inséré dans une membrane lipidique que l'on soumet à une différence de potentiel comprise entre -60 mV et-10 mV, dans un milieu réactionnel comprenant une solution d'électrolyte halogénure de métal alcalin ayant une concentration comprise entre 2M et 5M et une température comprise entre 3°C et - 33°C, et où la dite utilisation a pour objectif de différencier les dits peptides, protéines en fonction de leur longueur, de leur masse et de leur séquence.

2. Utilisation d'un nanopore selon la revendication 1, **caractérisée en ce que** l'halogénure de métal alcalin est un chlorure de métal alcalin.

3. Utilisation d'un nanopore selon la revendication 1 ou 2, **caractérisée en ce que** l'halogénure de métal alcalin est représenté par LiCl, KCl ou NaCl.

4. Utilisation d'un nanopore selon la revendication 3, **caractérisée en ce que** la concentration du milieu réactionnel en KCl est égale à 4M, et **en ce que** la température du milieu réactionnel est égale à 5°C.

5. Utilisation d'un nanopore selon l' 'une quelconque des revendications précédentes, **caractérisée en ce que** la différence de potentiel appliquée à la membrane est de -29 mV.

6. Utilisation d'un nanopore selon l'une quelconque des revendications 1 à 5 pour caractériser la dégradation enzymatique d'un échantillon de peptides, de protéines par détermination des produits de dégradation.

7. Utilisation d'un nanopore d'aérolysine selon la revendication 1 pour la détection électrique de peptides, de protéines dans un échantillon destiné à la médecine humaine, lesdits peptides et protéines étant distincts d'au moins un acide aminé dans une préparation où le dit nanopore est inséré dans une membrane lipidique que l'on soumet à une différence de potentiel de -29 mV, dans un milieu réactionnel comprenant une solution d'électrolyte de KCl ayant une concentration de 2M ou 4M et à une température de 20°C, et où la dite utilisation a pour objectif de différencier les dits peptides, protéines en fonction de leur longueur, de leur masse et de leur séquence.

## Patentansprüche

1. Verwendung einer Nanopore von Aerolysin zur elektrischen Detektion von Peptiden und Proteinen, welche sich durch mindestens eine Aminosäure in einem Präparat unterscheiden, in welchem die sogenannte Nanopore in eine Lipidmembran eingebracht ist, welche einer Potentialdifferenz, welche zwischen -60mV und -10mV liegt, in einem reaktiven Milieu ausgesetzt wird, welches eine Elektrolytlösung von Alkalimetallhalogenid umfasst, welches eine Konzentration zwischen 2M und 5M und eine Temperatur aufweist, welche zwischen 3°C und 33°C liegt, und wobei die sogenannte Verwendung zum Gegenstand hat, die sogenannten Peptide, Proteine entsprechend ihrer Länge, ihrer Masse und ihrer Reihenfolge zu unterscheiden.

2. Verwendung einer Nanopore nach Anspruch 1, **dadurch gekennzeichnet, dass** das Alkalimetallhalogenid ein Alkalimetallchlorid ist.

3. Verwendung einer Nanopore nach Anspruch 1 oder 2, **dadurch gekennzeichnet, dass** das Alkalimetallhalogenid durch LiCl, KCl oder NaCl dargestellt ist.

4. Verwendung einer Nanopore nach Anspruch 3, **dadurch gekennzeichnet, dass** die Konzentration des reaktiven Milieus aus KCl gleich 4M ist und dass die Temperatur des reaktiven Milieus gleich 5°C ist.

5. Verwendung einer Nanopore nach einem der vorhergehenden Ansprüche, **dadurch gekennzeichnet, dass** die an die Membran angelegte Potentialdifferenz -29mV beträgt.

6. Verwendung einer Nanopore nach einem der Ansprüche 1 bis 5, um den enzymatischen Abbau einer Probe von Peptiden, Proteinen durch Bestimmung der Abbauprodukte zu charakterisieren.

7. Verwendung einer Nanopore von Aerolysin nach Anspruch 1, zur elektrischen Detektion von Peptiden, Proteinen in einer Probe, welche für die Humanmedizin vorgesehen ist, wobei sich die sogenannten Peptide und Proteine durch mindestens eine Aminosäure in einem Präparat unterscheiden, in welchem die sogenannte Nanopore in eine Lipidmembran eingebracht ist, welche einer Potentialdifferenz von -29mV in einem reaktiven Milieu ausgesetzt wird, welches eine Elektrolytlösung von KCl mit einer Konzentration von 2M oder 4M und bei einer Temperatur von 20°C umfasst, und wobei die sogenannte Verwendung zum Gegenstand hat, die sogenannten Peptide, Proteine entsprechend ihrer Länge, ihrer Masse und ihrer Reihenfolge zu unterscheiden.

## Claims

1. The use of an aerolysin nanopore for the electrical detection of peptides, proteins separated by at least one amino acid in a preparation where said nanopore is inserted into a lipid membrane which is subjected to a difference in potential between -60mV and -10 mV, in a reaction medium comprising an alkali metal halide electrolyte solution with a concentration between 2M and 5M, and at a temperature between 3 °C and 33 °C, and where said use is intended to differentiate said peptides, proteins according to their length, their mass and their sequence.

2. Use of a nanopore according to Claim 1, wherein the alkali metal halide is an alkali metal chloride.

3. Use of a nanopore according to Claim 1 or 2, wherein the alkali metal halide is an alkali metal chloride represented by LiCl, KCl or NaCl.

4. Use of a nanopore according to Claim 3, wherein the concentration of the electrolyte solution in KCl is of 5M and the temperature of the reaction medium is equal to 5 °C.

5. Use of a nanopore according to any one of the preceding Claims, wherein the difference in potential applied to the membrane is of between -29 mV.

6. Use of a nanopore according to any one of Claims 1 to 5 to characterise the enzymatic degradation of a sample of peptides, proteins, by determination of the degradation products.

7. Use of an aerolysin nanopore according to Claim 1 for the electrical detection of peptides, proteins in a sample intended for human medicine, said peptides and proteins separated by at least one amino acid in a preparation where said nanopore is inserted into a lipid membrane which is subjected to a difference in potential between -29 mV, in a reaction medium comprising a KC1 electrolyte solution with a concentration of 2M or 4M, and at a temperature of 20 °C, and where said use is intended to differentiate said peptides, proteins according to their length, their mass and their sequence.
